# EUROPEAN PATENT APPLICATION

(11) **EP 4 484 548 A1**
(43) Date of publication of application: **01.01.2025**
(21) Application number: 23759874.3
(22) Date of filing: 17.02.2023
(51) Int. Cl.: C12N 1/20, C12P 9/00

(54) **LACTIC ACID BACTERIUM FOR SYNTHESIZING PLASMALOGEN, PLASMALOGEN-CONTAINING COMPOSITION, AND PLASMALOGEN PRODUCTION METHOD**

(30) Priority: 22.02.2022 JP 2022025310
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO, Takehiko, Fukuoka-shi, Fukuoka 813-0043 (JP); MAWATARI, Shiro, Fukuoka-shi, Fukuoka 813-0016 (JP); DOI, Katsumi, Fukuoka-shi, Fukuoka 819-0395 (JP); HONSHO, Masanori, Fukuoka-shi, Fukuoka 819-0395 (JP)
(74) Representative: V.O.
(86) International application number: PCT/JP2023/005656
(87) International publication number: WO 2023/162873

(57) **Abstract**

A lactic acid bacterium for plasmalogen synthesis synthesizes plasmalogens under aerobic conditions.

## Description

### Technical Field

The present disclosure relates to a lactic acid bacterium for plasmalogen synthesis, a plasmalogen-containing composition, and a plasmalogen production method.

### Background Art

Plasmalogens are a type of phospholipids having antioxidant activity and one of glycerophospholipids. Plasmalogens are present in all mammalian tissues and constitute approximately 18% of the phospholipids in the human body. In particular, plasmalogens are known to be abundant in cranial nerves, cardiac muscle, skeletal muscle, leukocytes, and sperm.

Since many plasmalogens are bound to polyunsaturated fatty acids such as docosahexaenoic acid and arachidonic acid, these plasmalogens are involved in storage of polyunsaturated fatty acids, release of secondary messengers for intercellular signaling such as prostaglandin and leukotriene produced from these polyunsaturated fatty acids, and the like. Plasmalogens are also involved in cell fusion, ion transport, and the like. Plasmalogens perform cellular antioxidant functions since the vinyl ether bonds (alkenyl bonds) of plasmalogens are particularly sensitive to oxidative stress.

Furthermore, plasmalogens are known to have an effect of accelerating neurogenesis, an inhibitory effect on neuroinflammation due to lipopolysaccharide (LPS), an inhibitory effect on the accumulation of cerebral amyloid β (Aβ) protein, and the like. For example, Patent Literature 1 discloses an anti-central nervous system inflammatory agent comprising plasmalogens.

Plasmalogens are known to be depleted in cranial nerve diseases such as dementia, Parkinson's disease, depression, and schizophrenia, as well as diabetes, metabolic syndrome, ischemic heart disease, infectious diseases, and immune disorders. For example, Non Patent Literature 1 reports significantly depleted serum ethanolamine-type plasmalogen levels in the frontal lobe and hippocampus in Alzheimer's disease brains. Furthermore, Non Patent Literature 2 reports depleted serum plasmalogen levels in Alzheimer's disease patients. Non Patent Literature 3 reports greater depletion of a choline-type plasmalogen in a group of patients with ischemic heart disease compared to that in a group of normal controls.

Through external replenishment of depleted plasmalogens, effects of preventing and ameliorating these diseases can be expected. Therefore, a demand for plasmalogens is predicted to increase in the future for the prevention and treatment of diseases, or maintenance of health.

Plasmalogens are widely present in invertebrates and vertebrates, but have not been identified in fungal and plant cells. Regarding bacteria, plasmalogen synthesis by some obligate anaerobes such as *Enterococcus faecalis* disclosed in Non-Patent Literature 4 and *Bifidobacterium longum* disclosed in Patent Literature 2 has been reported. However, no plasmalogen synthesis has been confirmed in facultative anaerobes and aerobic bacteria (see Non-Patent Literature 5).

### Citation List

### Patent Literature

Patent Literature 1: International Publication No. WO 2012/039472
Patent Literature 2: International Publication No. WO 2021/024872

### Non Patent Literature

Non Patent Literature 1: Z. Guan et al., Decrease and Structural Modifications of Phosphatidylethanolamine Plasmalogen in the Brain with Alzheimer Disease, J Neuropathol Exp Neurol, 58(7), 740-747, 1999
Non Patent Literature 2: D. B. Goodenowe et al., Peripheral ethanolamine plasmalogen deficiency: a logical causative factor in Alzheimer's disease and dementia, J Lipid Res, 48, 2485-2498, 2007
Non Patent Literature 3: Takeo SANADA et al., Serum plasmalogen dynamics in ischemic heart disease, Arteriosclerosis, 11, 535-539, 1983
Non Patent Literature 4: David R Jackson et al., Plasmalogen Biosynthesis by Anaerobic Bacteria: Identification of a Two-Gene Operon Responsible for Plasmalogen Production in Clostridium perfringens, ACS Chemical Biology, 11, 12, 3421-3430, 2021
Non Patent Literature 5: Howard Goldfine, The appearance, disappearance and reappearance of plasmalogens in evolution, Progress in Lipid Research, 49, 4, 493-498, 2010

### Summary of Invention

### Technical Problem

If plasmalogens can be biosynthesized by bacteria, plasmalogens can be obtained easily and in large quantities by culturing the bacteria. However, when plasmalogens are biosynthesized by obligate anaerobes as described above, it is necessary to culture the bacteria under anaerobic conditions. Culturing under anaerobic conditions requires equipment or the like that does not allow oxygen to enter, and especially when the scale of culturing is increased, maintaining anaerobic conditions becomes difficult and complicated.

The present disclosure has been achieved in view of the above circumstances, and an objective thereof is to provide a lactic acid bacterium for plasmalogen synthesis, a plasmalogen-containing composition, and a plasmalogen production method, with which plasmalogens can be conveniently obtained.

### Solution to Problem

The inventors of the present disclosure have screened various lactic acid bacteria, discovered that specific lactic acid bacteria synthesize plasmalogens under aerobic conditions, and thus completed the present disclosure.

A lactic acid bacterium for plasmalogen synthesis according to a first aspect of the present disclosure synthesizes a plasmalogen under an aerobic condition.

The lactic acid bacterium for plasmalogen synthesis according to the first aspect of the present disclosure may belong to a family Streptococceae, Lactobacillaceae, or Enterococcaceae.

A plasmalogen-containing composition according to a second aspect of the present disclosure comprises a cell of the lactic acid bacterium for plasmalogen synthesis according to the first aspect of the present disclosure or a processed product thereof.

A method for producing a plasmalogen according to a third aspect of the present disclosure comprises:
culturing the lactic acid bacterium for plasmalogen synthesis according to the first aspect of the present disclosure under an aerobic condition; and
obtaining a plasmalogen from a cell of the lactic acid bacterium for plasmalogen synthesis.

In the culturing, the lactic acid bacterium for plasmalogen synthesis may be statically cultured.

### Advantageous Effects of Invention

According to the present disclosure, plasmalogens can be conveniently obtained.

### Brief Description of Drawings

FIG. 1 illustrates chromatograms relating to the total lipids of each sample in Examples;
FIG. 2 illustrates chromatograms relating to aldehydes with respect to JCM7879, JCM5705 and JCM1166 in Examples; and
FIG. 3 illustrates chromatograms relating to aldehydes with respect to JCM8726 in Examples.

### Description of Embodiments

An embodiment according to the present disclosure is described with reference to the drawings. The present disclosure is not limited by the following embodiments and drawings. In the following embodiments, expressions such as "have", "comprise" or "include" and "contain" also include the meaning of "consisting of" or "composed of'.

### Embodiment 1

The lactic acid bacteria for plasmalogen synthesis according to the present embodiment synthesize plasmalogens under aerobic conditions. Aerobic conditions refer to conditions in which oxygen is contained in the atmosphere. Aerobic conditions also include microaerobic conditions. In particular, culturing under aerobic conditions refers to culturing that is performed without the use of reagents and equipment to be used for culturing microorganisms under anaerobic conditions.

Lactic acid bacteria for plasmalogen synthesis synthesize plasmalogens when cultured under aerobic conditions. The lactic acid bacteria for plasmalogen synthesis are preferably cultured at or near the optimum temperature, and the culture temperature ranges from, for example, 25°C to 40°C, 28°C to 38°C, or 30°C to 37°C. The lactic acid bacteria for plasmalogen synthesis can be cultured by a known method, for example, in a liquid medium or an agar medium. Preferably, the lactic acid bacteria for plasmalogen synthesis are statically cultured.

The lactic acid bacteria for plasmalogen synthesis are not particularly limited as long as they are lactic acid bacteria (Lactobacillales; bacteria belonging to the order Lactobacillus) that synthesize plasmalogens under aerobic conditions. The lactic acid bacteria for plasmalogen synthesis are bacteria belonging to, for example, the family Aerococcaceae, the family Carnobacteriaceae, the family Enterococcaceae, the family Streptococcaceae, and the family Lactobacillaceae. Preferably, the lactic acid bacteria for plasmalogen synthesis belong to the family Streptococcaceae, Lactobacillaceae or Enterococcaceae.

Specifically, examples of the lactic acid bacteria for plasmalogen synthesis include *Streptococcus equinus, Streptococcus mutans, Lactobacillus coryniformis,* and *Enterococcus faecalis.*

Plasmalogens belong to a unique subclass of glycerophospholipids characterized by having a vinyl ether bond at the sn-1 position and an ester bond at the sn-2 position of the glycerol backbone. The plasmalogens in the present embodiment are not particularly limited as long as they are glycerophospholipids generally classified as plasmalogens. Examples of the plasmalogens include an ethanolamine-type plasmalogen (Pls), a choline-type plasmalogen (PlsCho), an inositol-type plasmalogen, and a serine-type plasmalogen. Pls has a structure in which -CH₂CH₂NH₂ is bound to the oxygen atom that is bound to phosphorus at the sn-3 position of the glycerol backbone. PlsCho has a structure in which -CH₂CH₂N(CH₃)₃ is bound to the oxygen atom that is bound to phosphorus at the sn-3 position of the glycerol backbone. The plasmalogens synthesized by the lactic acid bacteria for plasmalogen synthesis according to the present embodiment have various numbers of carbon atoms constituting the carbon chain that is bound to the oxygen atom at the sn-1 position, and examples thereof include, in addition to plasmalogens in mammals, plasmalogens having the number of carbon atoms not seen among mammals.

The lactic acid bacteria for plasmalogen synthesis according to the present embodiment can synthesize plasmalogens even under aerobic conditions, as described in the examples below. Therefore, plasmalogens can be conveniently obtained without the need to maintain anaerobic conditions as a cell culture atmosphere.

### Embodiment 2

The plasmalogen-containing composition according to the present embodiment contains the cells of the lactic acid bacteria for plasmalogen synthesis according to Embodiment 1 above or a processed product thereof. The cells can be obtained as a precipitate by centrifugation of the culture solution after culturing.

Examples of the processed products of the cells include cells obtained by subjecting the cells to some treatments, such as freeze-dried cells, acetone-dried cells, extracts obtained by subjecting cells to solvent extraction, and disrupted products obtained by disrupting cells or dried cells with ultrasonic waves or the like, and compositions derived from the cells or compositions containing portions of the cells. As solvents to be used for solvent extraction, water, an organic solvent, and a mixture thereof can be used. Examples of organic solvents include ether, chloroform, benzene, hexane, methanol, ethanol, isopropanol, and mixed solutions thereof. An extract extracted using the extraction solvent can be used intact as a liquid, or after being concentrated or diluted, can be used in the form of liquid, gel, or paste. Furthermore, the resultant can be used as a dried dry product. Drying can be carried out by known methods such as spray drying, freeze drying, vacuum drying and fluidized drying.

The plasmalogen-containing composition according to the present embodiment contains a plasmalogen synthesized by a lactic acid bacterium for plasmalogen synthesis. Therefore, the plasmalogen-containing composition is useful for externally replenishing plasmalogens in living organisms. For example, the plasmalogen-containing composition is used to treat and prevent diseases in which plasmalogens are depleted. Examples of such diseases include inflammatory diseases, central nervous system inflammatory diseases, dementia, Parkinson's disease, depression, schizophrenia, diabetes, metabolic syndrome, ischemic heart disease, infectious diseases, and immune disorders.

In another embodiment, the plasmalogen-containing composition is used as a therapeutic or prophylactic agent for the above-exemplified plasmalogen-depleted diseases. In another embodiment, the plasmalogen-containing composition is used as a therapeutic or prophylactic agent for the above-exemplified plasmalogen-depleted diseases. In another embodiment, an oral composition for treating a disease in which plasmalogens are depleted is provided, containing the cells of the lactic acid bacterium for plasmalogen synthesis according to Embodiment 1 or a processed product thereof. Specific examples of the oral composition include supplements, food compositions, foods and drinks, functional foods, and food additives.

In another embodiment, a method for producing plasmalogens using the lactic acid bacterium for plasmalogen synthesis according to Embodiment 1 above is provided. The method for producing plasmalogens includes a culturing step and an obtaining step. In the culturing step, the lactic acid bacterium for plasmalogen synthesis is cultured under aerobic conditions, as described above. In the obtaining step, plasmalogens are obtained from the cells of the lactic acid bacterium for plasmalogen synthesis. In the obtaining step, plasmalogens can be obtained by treating the cells by solvent extraction, disruption via ultrasonic waves, or the like, as described above. The plasmalogens obtained in the obtaining step may be purified by a known method or isolated, or the purified plasmalogens may be concentrated.

### Examples

The present disclosure is described more specifically by the following Examples, but the present disclosure is not limited by Examples.

### [Culturing of lactic acid bacteria]

*Lactobacillus coriniformis* (JCM1166), *Streptococcus mutans* (JCM5705), *Streptococcus equinus* (JCM7879) and *Enterococcus faecalis* (JCM8726) were obtained as lactic acid bacteria from the Microbe Division in RIKEN BioResource Research Center (RIKEN BRC). Stocks of lactic acid bacteria were applied to plate media, and lactic acid bacterial colonies were cultured in liquid media.

A BL agar medium (manufactured by Eiken Chemical Co., Ltd.) was used as the plate medium for JCM1166. A TRYPTO-SOYA agar medium was used as the plate medium for JCM5705. A BHIY agar medium was used as the plate medium for JCM7879. An MRS agar medium was used as the plate medium for JCM8726. The composition of the TRYPTO-SOYA agar medium (pH 7.2) includes 15 g of peptone (Beco Pepton, manufactured by Becton, Dickinson and Company), 5 g of soybean peptone (Soytone, manufactured by Becton, Dickinson and Company), 5 g of sodium chloride (manufactured by NACALAI TESQUE, INC.), 15 g of agar and 1 L of distilled water. The composition of the BHIY agar medium (pH 7.4) includes 37 g of Brain heart infusion (manufactured by Becton, Dickinson and Company), 20 g of yeast extract (manufactured by Becton, Dickinson and Company), 15 g of agar, and 1 L of distilled water. The composition of the MRS agar medium (pH 6.5) includes 55 g of Lactobacilli MRS Broth (manufactured by Becton, Dickinson and Company), 20 g of agar and 1 L of distilled water.

For culturing in a liquid medium, an MRS medium was used as the liquid medium for JCM1166 and JCM8726. As the liquid medium for JCM5705, a TRYPTO-SOYA medium having a composition obtained by removing agar from the TRYPTO-SOYA agar medium was used. As the liquid medium for JCM7879, a BHIY medium having a composition obtained by removing agar from the above BHIY agar medium was used. All of the lactic acid bacteria were statically cultured in a 10 mL liquid medium for 12 hours while maintaining the temperature in an incubator. The culture temperatures for JCM1166 and JCM8726 were 30°C and 37°C, respectively, and the culture temperature for JCM5705 and JCM7879 was 35°C.

### [Lipid extraction from cells and detection of phospholipids]

The culture solution was centrifuged after culturing to recover the cells. Physiological saline was added to and mixed with the cells. After centrifugation, the supernatant was removed to wash the cells. Physiological saline was further added to the cells and washed again.

The total weight of the thawed cells was measured. After adding 1 mL of water to the cells and mixing, 3.75 mL of chloroform/methanol (1:2, v/v) was added and mixed. The resultant was sonicated for 10 minutes and left at room temperature for 30 minutes. 1.25 mL of chloroform was added and then 1.25 mL of water was added. After centrifugation, the chloroform layer was transferred to a glass tube and the remaining aqueous layer was re-extracted with 2 mL of chloroform. The combined chloroform layer was dried under nitrogen gas, resuspension was performed in hexane/isopropanol (3:2, v/v) in such a manner that the cell concentration was 120 mg/mL, followed by filtration through a 0.45 µm filter. 10 µL of the cell suspension was injected into high performance liquid chromatography (HPLC) and then subjected to detection with an evaporative light scattering detector (ELSD).

For comparison, lipids were also extracted as follows from human erythrocyte membranes as plasmalogens in mammals. 10 mL of human venous blood was collected in a heparinized blood collection tube and centrifuged at 4°C for 5 minutes (1,000×g (3,000 rpm in a clinical centrifuge)). The upper layer was removed, the erythrocyte layer was transferred to a 15 mL graduated plastic tube, and diluted to 10 mL with physiological saline to obtain a sample. Samples were each centrifuged (1,000×g) at 4°C for 5 minutes to remove the upper layer and 1 mm to 2 mm of the erythrocyte layer. The removal of the upper layer and the red blood cell layer after this centrifugation was repeated a total of three times, and then 1 mL of the erythrocyte layer was added to a 50 mL centrifuge tube. 30 mL of 10 mM Tris-HCl (pH 7.4-7.5) was mixed with the sample to hemolyze erythrocytes. The sample was centrifuged at 4°C for 15 minutes (10,000×g (12,000 rpm in a clinical centrifuge)), and then the red upper layer was removed. The removal of the upper layer after this centrifugation was repeated a total of three times, and then the upper layer was removed to obtain a white erythrocyte membrane.

To 100 µL of the obtained erythrocyte membrane solution, 1 mL of methanol containing dibutylhydroxytoluene (BHT) and 2 mL of chloroform containing BHT were added and mixed. The solution was left to stand at room temperature for 40 minutes. 0.6 mL of 50 mM KCl (0.88% KCl) was added to and mixed with the solution. The solution was centrifuged (1,000×g) at 20°C for 5 minutes and then 1 mL of the lower layer (chloroform layer) was transferred to another test tube. After drying the solution with nitrogen gas for solidification, the resultant was stored at -30°C. After redissolving with 200 µL of hexane isopropanol (3:2, v/v) and passing through a 0.45 µm filter, 10 µL of the resultant was used as a sample for HPLC.

As a lipid standard sample, phosphatidylcholine (25 mg, 5 mg/mL in chloroform, A-29B, manufactured by SRL) was dried with nitrogen gas for solidification, and then 1 mL of isopropanol/chloroform (2:1, v/v) was added and mixed. Samples were ultrasonically dissolved and stored as lipid stocks at -30°C. Each lipid stock was serially diluted with HIP (hexane/isopropanol (3:2, v/v)) to prepare a lipid standard solution. The lipid standard solution was filtered through a 0.45 µm filter, so as to obtain 10 µL of a sample for HPLC.

The HPLC conditions are as follows.
Column: Lichrosphere DIOL (250 × 3 mm, 5 µm, manufactured by Merck)
Mobile phase A: hexane/isopropanol/acetic acid (82:17:1) containing 0.08% triethylamine
Mobile phase B: isopropanol/water/acetic acid (85:14:1) containing 0.08% triethylamine
Flow rate: 0.8 mL/minutes
Column temperature: 50°C
Gradient:
   0 minutes mobile phase A 96% mobile phase B 4%
   21 minutes Mobile phase A 63% Mobile phase B 37%
   25 minutes Mobile phase A 15% Mobile phase B 85%
   26 minutes Mobile phase A 15% Mobile phase B 85%
   29 minutes Mobile phase A 96% Mobile phase B 4%
   34 minutes Mobile phase A 96% Mobile phase B 4%

The ELSD conditions are as follows.
Evaporator temperature: 60°C
Nebulizer temperature: 30°C
Nitrogen gas flow rate: 1.00 SLM

Lipid standard solutions with different concentrations were also subjected to detection by HPLC-ELSD. A calibration curve was prepared from the peak area at each concentration, and the amount of phospholipid was calculated from the peak area ratio corresponding to the phospholipids obtained from the lactic acid bacteria with a retention time of 10 to 20 minutes.

### [Changes in plasmalogen due to acid hydrolysis treatment]

To the lipids that had been extracted with chloroform/methanol from the cells and then dried with nitrogen gas as described above, 0.5 mL of 2,4-dinitrophenylhydrazine-hydrochloride solution (DNPH-HCl solution, manufactured by Tokyo Chemical Industry Co., Ltd.) was added and mixed therewith. After leaving the resultant at room temperature for 30 minutes, 0.5 mL of ultrapure water was added to and mixed with the acid-hydrolyzed lipids, and then 1.9 mL of chloroform/methanol (1:2, v/v) was added to and mixed therewith. After leaving the resultant at room temperature for 10 minutes, 0.625 mL of chloroform was added. Subsequently, 0.625 mL of water was added. After centrifugation at 3,000 rpm and room temperature, the chloroform layer was transferred to a glass tube and then dried under nitrogen gas. Dried lipids were resuspended in 100 µL of acetonitrile and then passed through a 0.45 µm filter. As a sample for HPLC, 10 µL of the resultant was injected into HPLC and then subjected to detection with ultraviolet rays (UV) at a wavelength of 356 nm.

An aldehyde standard solution used as a standard solution was prepared as follows. Decanal (C₁₀H₂₀O, manufactured by Tokyo Chemical Industry Co., Ltd., D0032), dodecanal (C₁₂H₂₄O, manufactured by Tokyo Chemical Industry Co., Ltd., D0979), tetradecanol (C₁₄H₂₈O, manufactured by Tokyo Chemical Industry Co., Ltd., T2696), hexadecanol (C₁₆H₃₂O, manufactured by Tokyo Chemical Industry Co., Ltd., H1296), heptadecanol (C₁₇H₃₄O, manufactured by Tokyo Chemical Industry Co., Ltd., H1295) and octadecanol (C₁₈H₃₆O, manufactured by Tokyo Kasei Kogyo Co., Ltd. O0368) were separately placed in spitz tubes and adjusted to 1 mg/mL with acetonitrile. 200 µL of the prepared 1 mg/mL solution (100 µL, 200 µL and 400 µL in the case of C₁₄H₂₈O alone) was placed in each spitz tube, and then 0.5 mL of a DNPH-HCl solution was added to and mixed with the sample dried with nitrogen gas for solidification. Ultrasonic emulsification was performed and the sample was left at room temperature for 30 minutes. 0.5 mL of ultrapure water and 1.9 mL of chloroform/methanol (1:2, v/v) were added to the sample, followed by mixing by vortexing. After leaving the resultant at room temperature for 10 minutes, 0.625 mL of chloroform was added to and mixed with the resultant by vortexing. 0.625 mL of ultrapure water was added to the sample and then mixed by vortexing. The sample was centrifuged (3,000 rpm) for 5 minutes at room temperature. The lower layer was transferred to another spitz tube, dried with nitrogen gas for solidification, dissolved in 2 mL of acetonitrile, and then stored at -30°C as a stock aldehyde standard solution.

120 µL of the stock aldehyde standard solution of C₁₄H₂₈O was taken at each concentration, filtered through a 0.45 µm filter, and then 10 µL of the solution was injected into HPLC. In order to prepare a mixed aldehyde standard solution in which 4 or 6 types of aldehydes were mixed, 30 µL each of 4 or 6 types of stock aldehyde standard solutions was taken and then mixed. The resulting mixed aldehyde standard solution was filtered through a 0.45 µm filter and then 10 µL of the solution was injected into HPLC.

The HPLC conditions are as follows.
Column: XBridge BEA C18 (3.0×150, 2.5 µm, manufactured by Waters)
Column temperature: 40°C
Mobile phase A: Acetonitrile
Mobile phase B: Water
Flow rate: 0.3 mL/minutes
Column temperature: 40°C
Gradient:
   0 minutes Mobile phase A 30% Mobile phase B 70%
   25 minutes Mobile phase A 100% Mobile phase B 0%
   40 minutes Mobile phase A 100% Mobile phase B 0%
   40.1 minutes Mobile phase A 30% Mobile phase B 70%
   50 minutes Mobile phase A 30% Mobile phase B 70%

### (Result)

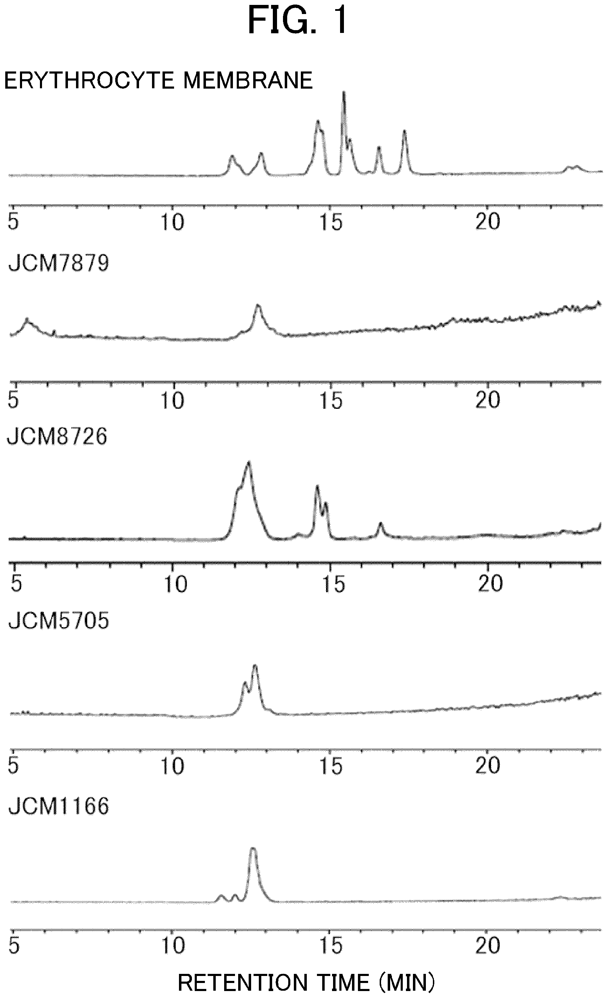

FIG. 1 illustrates the chromatogram of each sample after detection by ELSD. The weight of total lipids was determined from the chromatogram using the standard curve.

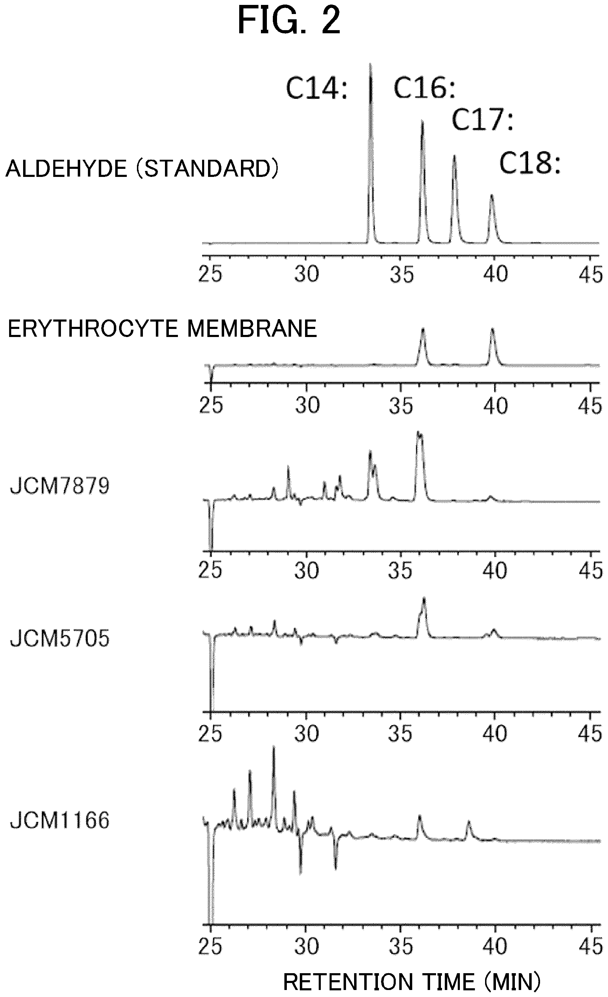

FIG. 2 illustrates the chromatograms of JCM7879, JCM5705 and JCM1166 treated with a DNPH-HCl solution. The top row in FIG. 2 is the aldehyde chromatogram indicating the retention times of C₁₄H₂₈O (aldehyde C14), C₁₆H₃₂O (aldehyde C16), C₁₇H₃₄O (aldehyde C17) and C₁₈H₃₆O (aldehyde C18). When phospholipids contained in total lipids were treated with a DNPH-HCl solution and then aldehyde was detected, this indicates the presence of plasmalogens in the phospholipids. Generation of aldehyde C16 and aldehyde C18 was confirmed in the total lipids of the erythrocyte membrane.

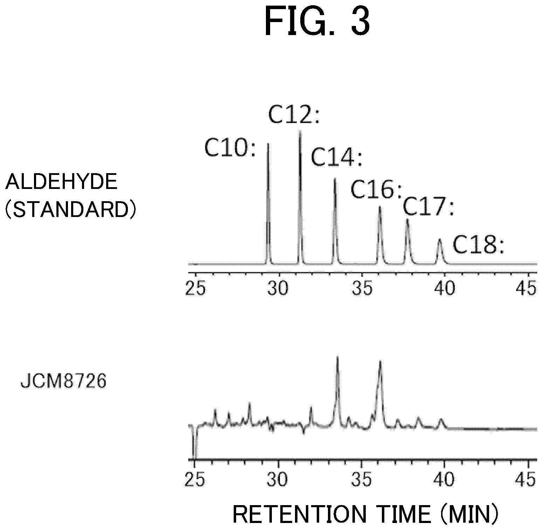

FIG. 3 illustrates the chromatogram of JCM8726 treated with a DNPH-HCl solution. The top row of FIG. 3 is the aldehyde chromatogram indicating the retention times of C₁₀H₂₀O (aldehyde C10) and C₁₂H₂₄O (aldehyde C12) in addition to aldehyde C14, aldehyde C16, aldehyde C17 and aldehyde C18.

Aldehydes were detected in all lactic acid bacteria, indicating that these lactic acid bacteria contain plasmalogens. At least aldehyde C14 and aldehyde C16 were detected in JCM7879 and JCM5705, and at least aldehyde C16 and aldehyde C17 were detected in JCM1166. At least aldehyde C14, aldehyde C16 and aldehyde C18 were detected in JCM8726. In the chromatograms of the lactic acid bacteria, aldehydes were also detected at retention times different from those in the chromatogram of the erythrocyte membrane. This suggests that plasmalogens produced by lactic acid bacteria include plasmalogens that are different from plasmalogens found in mammals.

Table 1 lists the weights of phospholipids and aldehydes per mg of the wet weight of the cells of lactic acid bacteria obtained from the calibration curve.

**Table 1**

| | Weight (µg) per mg of wet weight of cells | | Aldehyde/Phospholipid (%) |
|---|---|---|---|
| | Phospholipid | Aldehyde | |
| JCM7879 | 0.53 | 0.12 | 22.6 |
| JCM5705 | 0.52 | 0.06 | 11.7 |
| JCM1166 | 0.78 | 0.04 | 5.2 |
| JCM8726 | 1.02 | 0.07 | 6.4 |

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-25310, filed on February 22, 2022, the entire disclosure of which is incorporated by reference herein.

### Industrial Applicability

The present disclosure is useful for producing plasmalogens.

## Claims

1. A lactic acid bacterium for plasmalogen synthesis, synthesizing a plasmalogen under an aerobic condition.

2. The lactic acid bacterium for plasmalogen synthesis according to claim 1, wherein
lactic acid bacterium belongs to a family Streptococcaceae, Lactobacillaceae or Enterococcaceae.

3. A plasmalogen-containing composition, comprising:
a cell of the lactic acid bacterium for plasmalogen synthesis according to claim 1 or 2, or a processed product thereof.

4. A method for producing a plasmalogen, the method comprising:
culturing the lactic acid bacterium for plasmalogen synthesis according to claim 1 or 2 under an aerobic condition; and
obtaining a plasmalogen from a cell of the lactic acid bacterium.

5. The method for producing a plasmalogen according to claim 4, wherein in the culturing, the lactic acid bacterium is statically cultured.
